# EUROPEAN PATENT SPECIFICATION

(11) **EP 2 419 086 B1**
(45) Date of publication and mention of the grant of the patent: **26.06.2013**
(21) Application number: 10714413.1
(22) Date of filing: 12.04.2010
(51) Int. Cl.: A61K 9/14

(54) **BIOAVAILABLE COMPOSITIONS OF AMORPHOUS ALPHA-(N-SULFONAMIDO)ACETAMIDE COMPOUND**
BIOVERFÜGBARE ZUSAMMENSETZUNGEN VON AMORPHEN ALPHA-(N-SULFONAMIDO)ACETAMID-VERBINDUNGEN
COMPOSITIONS BIODISPONIBLES DE COMPOSÉ ALPHA-(N-SULFONAMIDO)ACÉTAMIDE AMORPHE

(30) Priority: 14.04.2009 US 169066 P
(43) Date of publication of application: 22.02.2012
(73) Proprietor: Bristol-Myers Squibb Company, Princeton, NJ 05843-4000 (US)
(72) Inventor: HARTLEY, Ruiling, F., New Brunswick, New Jersey 08903 (US); HADDADIN, Raja, M., New Brunswick, New Jersey 08903 (US); QIAN, Feng, New Brunswick, New Jersey 08903 (US)
(74) Representative: Reitstötter - Kinzebach
(86) International application number: PCT/US2010/030679
(87) International publication number: WO 2010/120662

(56) References cited:
- WO-A1-03/053912
- WO-A1-2009/058552

## Description

### FIELD OF THE INVENTION

The present invention relates to pharmaceutical formulations containing the Beta amyloid peptide production inhibitor compound (2R)-2-[[(4-chlorophenyl)sulfonyl][[2-fluoro-4-(1,2,4-oxadiazol-3-yl)phenyl]methyl]amino]-5,5,5-trifluoropentanamide, and more particularly, to compositions that are storage stable for extended periods and are orally bioavailable containing (2R)-2-[[(4-chlorophenyl) sulfonyl] [[2-fluoro-4-(1,2,4-oxadiazol-3-yl)phenyl]methyl]amino]-5,5,5-trifluoropentanamide together with one or more pharmaceutically acceptable polymers.

### BACKGROUND OF THE INVENTION

Alzheimer's disease (AD) is a progressive neurodegenerative disease which begins with memory loss and progresses to include severe cognitive impairment, altered behavior, and decreased motor function (Grundman, M. et al., Arch Neurol., 61:59-66 (2004); Walsh, D.M. et al., Neuron, 44:181-193 (2004)). It is the most common form of dementia and represents the third leading cause of death after cardiovascular disorders and cancer. The cost of AD is enormous and includes the suffering of the patients and families and the lost productivity of patients and caregivers. No treatment that effectively prevents AD or reverses the clinical symptoms and underlying pathophysiology is currently available. A definitive diagnosis of AD for a demented patient requires a histopathological evaluation of the number and localization of neuritic plaques and neurofibrillary tangles upon autopsy (Consensus recommendations for the postmortem diagnosis of Alzheimer's disease. Neurobiol. Aging, 18:S1-S2 (1997)). Similar alterations are observed in patients with Trisomy 21 (Down syndrome). Plaques primarily consist of β-amyloid (Aβ) peptides that are formed by a stepwise proteolytic cleavage of the amyloid precursor protein (APP) by β-site APP-cleaving enzyme (BACE), to generate the N-terminus, and γ-secretase, to generate the C-terminus (Selkoe, D.J., Physiol. Rev., 81:741-766 (2001)). γ-Secretase is a transmembrane protein complex that includes Nicastrin, Aph-1, PEN-2, and either Presenilin-1 (PS-1) or Presenilin-2 (PS-2) (Wolfe, M.S. et al., Science, 305:1119-1123 (2004)). PS-1 and PS-2 are believed to contain the catalytic sites of γ-secretase.

Aβ40 is the most abundant form of Aβ synthesized (80-90%), while Aβ42 is most closely linked with AD pathogenesis. In particular, mutations in the APP, PS-1, and PS-2 genes that lead to rare, familial forms of AD implicate Aβ42 aggregates as the primary toxic species (Selkoe, D.J., Physiol Rev., 81:741-766 (2001)). Current evidence suggests that oligomeric, protofibrillar and intracellular Aβ42 play a significant role in the disease process (Cleary, J.P. et al., Nat. Neurosci., 8:79-84 (2005)). Inhibitors of the enzymes that form Aβ42, such as γ-secretase, represent potential disease-modifying therapeutics for the treatment of AD. γ-Secretase cleaves multiple type I transmembrane proteins in addition to APP (Pollack, S.J. et al., Curr. Opin. Invest. Drugs, 6:35-47 (2005)), While the physiological significance of most of these cleavage events is unknown, genetic evidence indicates that γ-secretase cleavage of Notch is required for Notch signaling (Artavanis-Tsakonas, S. et al., Science, 284(5415):770-776 (1999); Kadesch, T., Exp. Cell Res., 260(1):1-8 (2000)). In rodents dosed with γ-secretase inhibitors, drug-related toxicity has been identified in the gastrointestinal (GI) tract, thymus, and spleen (Searfoss, G.H. et al., J. Biol. Chem., 278:46107-46116 (2003); Wong, G.T. et al., J. Biol. Chem., 279:12876-12882 (2004); Milano, J. et al., Toxicol. Sci., 82:341-358 (2004)). These toxicities are likely linked to inhibition of Notch signaling (Jensen, J. et al., Nat. Genet., 24:36-44 (2000)).

The identification of mechanism-based toxicity raises the question of whether an acceptable therapeutic index can be achieved with γ-secretase inhibitors. Selective inhibition of Aβ formation over Notch processing, pharmacokinetics, drug disposition and/or tissue-specific pharmacodynamics could impact therapeutic margin.

Evidence suggests that a reduction in brain Aβ levels by inhibition of γ-secretase may prevent the onset and progression of AD (Selkoe, D., Physiol. Rev., 81:741-766 (2001); Wolfe, M., J. Med. Chem., 44:2039-2060 (2001)). There are emerging data for the role of Aβ in other diseases, including mild cognitive impairment (MCI), Down syndrome, cerebral amyloid angiopathy (CAA), dementia with Lewy bodies (DLB), amyotrophic lateral sclerosis (ALS-D), inclusion body myositis (IBM), and age-related macular degeneration. Advantageously, compounds that inhibit γ-secretase and reduce production of Aβ could be used to treat these or other Aβ-dependent diseases.

Excess production and/or reduced clearance of Aβ causes CAA (Thal, D. et al., J. Neuropath. Exp..Neuro., 61:282-293 (2002)). In these patients, vascular amyloid deposits cause degeneration of vessel walls and aneurysms that may be responsible for 10-15% of hemorrhagic strokes in elderly patients. As in AD, mutations in the gene encoding Aβ lead to an early onset form of CAA, referred to as cerebral hemorrhage with amyloidosis of the Dutch type, and mice expressing this mutant protein develop CAA that is similar to patients. Compounds that specifically target γ-secretase could reduce or prevent CAA.

DLB manifests with visual hallucinations, delusions, and parkinsonism. Interestingly, familial AD mutations that cause Aβ deposits can also cause Lewy bodies and DLB symptoms (Yokota, O. et al., Acta Neuropathol. (Berl.), 104:637-648 (2002)). Further, sporadic DLB patients have Aβ deposits similar to those in AD (Deramecourt, V. et al., J. Neuropathol. Exp. Neurol., 65:278-288 (2006)). Based on this data, Aβ likely drives Lewy body pathology in DLB and, therefore, γ-secretase inhibitors could reduce or prevent DLB.

Approximately 25% of ALS patients have significant dementia or aphasia (Hamilton, R.L. et al., Acta Neuropathol. (Berl.), 107:515-522 (2004)). The majority (∼60%) of these patients, designated ALS-D, contain ubiquitin-positive inclusions comprised primarily of the TDP-43 protein (Neumann, M. et al., Science, 314:130-133 (2006)). About 30% of the ALS-D patients have amyloid plaques consistent with Aβ causing their dementia (Hamilton, R.L. et al., Acta Neuropathol. (Berl.), 107:5 5-522 (2004)). These patients should be identifiable with amyloid imaging agents and potentially treatable with γ-secretase inhibitors.

IBM is a rare, age-related degenerative disease of skeletal muscle. The appearance of Aβ deposits in IBM muscle and the recapitulation of several aspects of the disease by directing APP overexpression to muscle in transgenic mice support the role of Aβ in IBM (reviewed in Murphy, M.P. et al., Neurology, 66:S65-S68 (2006)). Compounds that specifically target γ-secretase could reduce or prevent IBM. In age-related macular degeneration, Aβ was identified as one of several components of drusen, extracellular deposits beneath the retinal pigment epithelium (RPE) (Anderson, D.H. et al., Exp. Eye Res., 78:243-256 (2004)). A recent study has shown potential links between Aβ and macular degeneration in mice (Yoshida, T. et al., J. Clin. Invest., 115:2793-2800 (2005)). Increases in Aβ deposition and supranuclear cataracts have been found in AD patients (Goldstein, L.E. et al., Lancet, 361:1258-1265 (2003)). Compounds that specifically target γ-secretase could reduce or prevent age-related macular degeneration.

Based on the role of Notch signaling in tumorigenesis, compounds which inhibit γ-secretase may also be useful as therapeutic agents for the treatment of cancer (Shih, I.-M. et al., Cancer Res., 67:1879-1882 (2007)).

Compounds which inhibit gamma secretase may also be useful in treating conditions associated with loss of myelination, for example, multiple sclerosis (Watkins, T.A. et al., Neuron, 60:555-569 (2008)).

A recent study by Georgetown University Medical Center researchers suggests that gamma-secretase inhibitors may prevent long-term damage from traumatic brain injury (Loane, D.J., et al., Nat. Med., 1-3 (2009)). Smith et al. in International Application No. WO 00150391, published August 31, 2000, disclose a series of sulfonamide compounds that can act to modulate production of amyloid β protein as a means of treating a variety of diseases, especially Alzheimer's disease and other diseases relating to the deposition of amyloid.
Japanese Patent No.11343279, published December 14, 1999 discloses a series of sulfonamide derivatives which are TNF-alpha inhibitors useful for treating autoimmune diseases.

Parker et al. in International Application No. WO 03/053912, published July 3, 2003, disclose a series of α-(N-sulphonamido)acetamide derivatives as β-amyloid inhibitors which are useful for the treatment of Alzheimer's disease and other conditions associated with β-amyloid peptide.

It has now been further discovered that an α-(N-sulphonamido)acetamide compound known as (2R)-2-[[(4-chlorophenyl)sulfonyl][[2-fluoro-4-(1,2,4-oxadfazol-3-yl)phenyl]methyl]amino]-5,5,5-trifluoropentanamide possesses unique attributes which make it useful for the treatment of Alzheimer's disease and other conditions associated with β-amyloid peptide. This compound is set forth and described in co-pending application with U.S. Patent Application Serial No. 12/249,180, filed October 10, 2008, the contents of which are incorporated herein in their entirety.

Unfortunately, (2R)-2-[[(4-chlorophenyl)sulfonyl][[2-fluoro-4-(1,2,4-oxadiazol-3-yl)phenyl]methyl]amino]-5,5,5-trifluoropentanamide has poor aqueous solubility that is often characterized as < 1 ug/mL at about room temperature. Moreover, there has been shown no appreciable improvement in bioavailability by particle size reduction. In addition, solid dosage forms containing the drug compound in a crystalline form showed low oral bioavailability in dogs. Thus, it now appears that in order to provide optimal exposure of the API, an amorphous (non-crystalline) form of the active compound should be provided.

What is therefore now needed in the art is one or more amorphous solid dispersion formulations containing the active compound (2R)-2-[[(4-chlorophenyl)sulfonyl][[2-fluoro-4-(1,2,4-oxadiazol-3-yl)phenyl]methyl]amino]-5,5,5-trifluoropentanamide, including pharmaceutically acceptable salts thereof, together with one or more pharmaceutically acceptable polymers, which may then be further utilized for forming tablets containing the active compound.

### SUMMARY OF THE INVENTION

In one embodiment, the present invention is directed to an amorphous solid dispersion composition comprising (2R)-2-[[(4-chlorophenyl)sulfonyl][[2-fluoro-4-(1,2,4-oxadiazol-3-yl)phenyl]methyl]amino]-5,5,5-trifluoropentanamide, and PVP-VA. As that term is used herein, "PVP-VA" refers to polyvinylpyrrolidone-vinyl acetate copolymer.

In another embodiment of the invention, there is provided an amorphous solid dispersion composition comprising (2R)-2-[[(4-chlorophenyl)sulfonyl][[2-fluoro-4-(1,2,4-oxadiazol-3-yl)phenyl]methyl]amino]-5,5,5-trifluoropentanamide, and HPMC-AS-AS. As that term is used herein, "HPMC-AS-AS" refers to hydroxypropylmethylcellulose acetate succinate (or hypromellose acetate succinate). Also provided herein is a pharmaceutical tablet containing an amorphous solid dispersion composition comprising (2R)-2-[[(4-chlorophenyl)sulfonyl][[2-fluoro-4-(1,2,4-oxadiazol-3-yl)phenyl]methyl]amino]-5,5,5-trifluoropentanamide and one member selected from the group consisting of PVP-VA and HPMC-AS-AS.

There is also provided methods of preparing the compositions of the invention by either hot-melt extrusion or spray drying.

In a further embodiment of the invention there is provided a method of treating or delaying the onset of Alzheimer's disease, cerebral amyloid angiopathy, mild cognitive impairment and/or Down syndrome, as well as the treatment of head trauma, traumatic brain injury, and/or dementia pugilistica, which comprises administering to a patient a therapeutically effective amount of a pharmaceutical tablet according to one or more of the embodiments hereinabove described. The present invention is directed to these, as well as other important ends, hereinafter described.

### DETAILED DESCRIPTION OF THE INVENTION

(2R)-2-[[(4-chlorophenyl)sulfonyl][[2-fluoro-4-(1,2,4-oxadiazol-3-yl)phenyl]methyl]amino]-5,5,5-trifluoropentanamide having the Formula I, including its pharmaceutically acceptable salts thereof, has now been found useful in inhibiting Aβ production in patients suffering from or susceptible to Alzheimer's disease (AD) or other disorders associated with β-amyloid peptide.

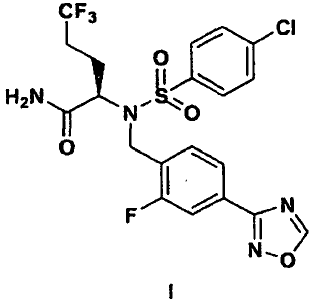

This compound has the chemical formula C₂₀H₁₇ClF₄N₄O₄S, and a molecular weight of 520.88. It is preferred that this compound be in the amorphous state. Since this compound has poor aqueous solubility, then it is now proposed to formulate it using PVP-VA according to one embodiment. PVP-VA can be represented by the structural formula below: 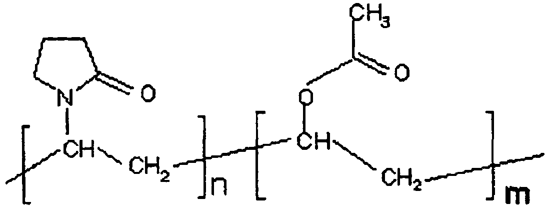 which represents the copolymer of polyvinylpyrrolidone and vinyl acetate. In the composition of the invention according to a first embodiment, there is provided about 10-50 w/v % of the amorphous compound (2R)-2-[[(4-chlorophenyl)sulfonyl][[2-fluoro-4-(1,2,4-oxadiazol-3-yl)phenyl]methyl]amino]-5,5,5-trifluoropentanamide, together with about 90-50 w/v % of PVP-VA. More preferably, there is provided about 20-40 w/v % of (2R)-2-[[(4-chlorophenyl)sulfonyl][[2-fluoro-4-(1,2,4-oxadiazol-3-yl)phenyllmethyl]amino]-5,5,5-trifluoropentanamide, together with about 80-60 w/v % of PVP-VA.

Even more preferably, the formulation of the invention contains about 25 w/v% of the active compound (2R)-2-[[(4-chlorophenyl)sulfonyl][[2-fluoro-4 oxadiazol-3-yl)phenyl]methyl]amino]-5,5,5-trifluoropentanamide, and also comprises about 75 w/v% of PVP-VA. Other excipients such as pharmaceutical-grade fillers and binders available in the art may also be incorporated therein the composition, but this is optional.

In order to prepare the compositions hereinabove described, various preparation means available to the skilled artisan may be utilized. It is preferred that the compositions containing the active compound and PVP-VA be prepared by the methods of hot-melt extrusion or spray-drying, with hot-melt extrusion being more preferred. A processing temperature of about 150-160°C is preferred for hot-melt extrusion processing of the active compound using apparatus available in the art. At a temperature of about 100°C, the compound (2R)-2-[[(4-chlorophenyl)sulfonyl][[2-fluoro-4-(1,2,4-oxadiazol-3-yl)phenyl]methyl]amino]-5,5,5-trifluoropentanamide has been shown to be less than 5% soluble in PVP-VA.

Thereafter the hot-melt extrusion processing, the extrudate so prepared may then be milled using equipment and procedures available in the art. It is preferred that particle size be reduced to about D₉₀ < 50 um.

In a further embodiment of the invention, there is provided a composition containing the compound (2R)-2-[[(4-chlorophenyl)sulfonyl][[2-fluoro-4-(1,2,4-oxadiazol-3-yl)phenyl]methyl]amino]-5,5,5-trifluoropentanamide, together with HPMC-AS. HPMC-AS is a polymer known as hydroxypropylmethylcellulose acetate succinate. Preferably, there is provided about 10-50 w/v % of the amorphous compound (2R)-2-[[(4-chlorophenyl)sulfonyl][[2-fluoro-4-(1,2,4-oxadiazol-3-yl)phenyl]methyl]amino]-5,5,5-trifluoropentanamide, together with about 90-50 w/v % of HPMC-AS. More preferably, there is provided about 20-40 w/v % of (2R)-2-[[(4-chlorophenyl)sulfonyl][[2-fluoro-4-(1,2,4-oxadiazol-3-yl)phenyl]methyl]amino]-5,5,5-trifluoropentanamide, together with about 80-60 w/v % of HPMC-AS.

Even more preferably, the composition of the invention contains about 25 w/v% of the active compound (2R)-2-[[(4-chlorophenyl)sulfonyl][[2-fluoro-4-(1,2,4-oxadiazol-3-yl)phenyl]methyl]amino]-5,5,5-trifluoropentanamide, together with about 75 w/v% of HPMC-AS. Other excipients such as pharmaceutical-grade fillers and binders available in the art may also be incorporated therein the composition, but this is optional.

In order to prepare the compositions hereinabove described containing (2R)-2-[[(4-chlorophenyl)sulfonyl][[2-fluoro-4-(1,2,4-oxadiazol-3-yl)phenyl]methyl]amino]-5,5,5-trifluoropentanamide and HPMC-AS, it is preferred to utilize spray drying procedures available in the art using acetone or methanol solution (∼ 5-10 % w/v). The inlet temperature of the spray dry apparatus is typically about 60-100°C, while the outlet temperature is about 30-60°C. The spray-dried material typically has particle size (D90) under 50 µm and therefore no further milling is required; however, the spray-dried material may be milled, if desired.

The compositions of the invention containing (2R)-2-[[(4-chlorophenyl)sulfonyl][[2-fluoro-4-(1,2,4-oxadiazol-3-yl)phenyl]methyl]amino]-5,5,5-trifluoropentanamide, together with PVP-VA or HPMC-AS, are highly storage stable. This means that they exhibit no crystallization by powder x-ray diffraction (PXRD) or differential scanning calorimeter (DSC) for at least about 3 months, and preferably for at least about 6 months, and even more preferably for at least about 9 months, when stored in a closed or open container under approximately 25°C/60% relative humidity, or in a closed container under approximately 40°C/75% relative humidity.

The compositions of the invention herein described according to the various embodiments may then be tabletted using equipment and procedures available in the art.

Furthermore, when desired or necessary, suitable additional binders, lubricants, disintegrating agents, and coloring agents can also be incorporated into the tabletting mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, and the like. Lubricants used in these dosage forms include sodium oleate, sodium chloride, and the like. Disintegrators include, without limitation, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

By way of non-limiting example, tablets containing about 50 mg. of the active compound (2R)-2-[[(4-chlorophenyl)sulfonyl][[2-fluoro-4-(1,2,4-oxadiazol-3-yl)phenyl]methyl]amino]-5,5,5-trifluoropentanamide may be made using the compositions herein described. Other dosage units are within the scope hereof. In particular, tablets containing cryo-milled (2R)-2-[[(4-chlorophenyl)sulfonyl][[2-fluoro-4-(1,2,4-oxadiazol-3-yl)phenyl]methyl]amino]-5,5,5-trifluoropentanamide and PVP-VA have demonstrated improved *in vitro* dissolution rates, and oral bioavailability in dogs that is similar to, that from a comparative solubilized capsule formulation. Similarly, a spray-dried composition containing (2R)-2-[[(4-chlorophenyl)sulfonyl][[2-fluoro-4-(1,2,4-oxadiazol-3-yl)phenyl]methyl]amino]-5,5,5-trifluoropentanamide and HPMC-AS also has shown improved *in vitro* dissolution rates, good *in vivo* oral bioavailability in dogs, and good chemical/physical stability.

In a further embodiment of the invention there is provided a method of treating or delaying the onset of Alzheimer's disease, cerebral amyloid angiopathy, mild cognitive impairment and/or Down syndrome, as well as the treatment of head trauma, traumatic brain injury, and/or dementia pugilistica, which comprises administering to a patient a therapeutically effective amount of a pharmaceutical tablet according to one or more of the embodiments hereinabove described. There is also provided a method of treating Alzheimer's disease in a patient, comprising administering to the patient a therapeutically effective amount of a pharmaceutical tablet according to one or more of the embodiments hereinabove described. Further provided is a method of inhibiting the functioning of a γ-secretase enzyme comprising contacting the γ-secretase enzyme with an effective amount of a pharmaceutical tablet according to one or more of the embodiments hereinabove described. Also provided is a method of inhibiting the production of β-amyloid peptide in a patient, comprising contacting a γ-secretase enzyme in the patient with an effective amount of a pharmaceutical tablet according to one or more of the embodiments hereinabove described. Further, a method of inhibiting the production of β-amyloid peptide in a patient comprises administering to the patient a therapeutically effective amount of a pharmaceutical tablet according to one or more of the embodiments hereinabove described. The term "therapeutically effective amount" means the total amount of the active component of the method that is sufficient to show a patient benefit, *i.e*., symptomatic or disease modifying treatment. When applied to an individual active ingredient, administered alone, the term refers to that ingredient alone. When applied to a combination, the term refers to combined amounts of the active ingredients that result in the therapeutic effect, whether administered in combination, serially or simultaneously.

## Claims

1. An amorphous solid dispersion composition comprising (2R)-2-[[(4-chlorophenyl)sulfonyl][[2-fluoro-4-(1,2,4-oxadiazol-3-yl)phenyl]methyl]amino]-5,5,5-trifluoropentanamide and polyvinylpyrrolidone-vinyl acetate copolymer (PVP-VA).

2. The composition of claim 1, comprising 10-50 w/v% of (2R)-2-[[(4-chlorophenyl)sulfonyl][[2-fluoro-4-(1,2,4-oxadiazol-3-yl)phenyl]methyl]amino]-5,5,5-trifluoropentanamide, and 90-50 w/v% of PVP-VA.

3. The composition of claim 2, comprising 20-40 w/v% of (2R)-2-[[(4-chlorophenyl)sulfonyl][[2-fluoro-4-(1,2,4-oxadiazol-3-yl)phenyl]methyl]amino]-5,5,5-trifluoropentanamide, and 80-60 w/v% of PVP-VA.

4. The composition of claim 3, comprising 25 w/v% of (2R)-2-[[(4-chlorophenyl)sulfonyl][[2-fluoro-4-(1,2,4-oxadiazol-3-yl)phenyl]methyl]amino]-5,5,5-trifluoropentanamide, and 75% of PVP-VA.

5. The composition of claim 1, wherein said composition is physically and chemically stable for at least 3 months.

6. The composition of claim 5, wherein said composition is physically and chemically stable for at least 6 months.

7. The composition of claim 1, wherein said composition is prepared by hot-melt extrusion or spray-drying.

8. The composition of claim 7, wherein said composition is tabletted.

9. The composition of claim 8, wherein said composition is orally bioavailable.

10. The composition of claim 1, wherein said composition is orally bioavailable.

11. An amorphous solid dispersion composition comprising (2R)-2-[[(4-chlorophenyl)sulfonyl][[2-fluoro-4-(1,2,4-oxadiazol-3-yl)phenyl]methyl]amino]-5,5,5-trifluoropentanamide and hydroxypropylmethylcellulose acetate succinate (HPMC-AS).

12. The composition of claim 11, comprising 10-50 w/v% of (2R)-2-[[(4-chlorophenyl)sulfonyl][[2-fluoro-4-(1,2,4-oxadiazol-3-yl)phenyl]methyl]amino]-5,5,5-trifluoropentanamide, and 90-50 w/v% of HPMC-AS.

13. The composition of claim 12, comprising 20-40 w/v% of (2R)-2-[[(4-chlorophenyl)sulfonyl][[2-fluoro-4-(1,2,4-oxadiazol-3-yl)phenyl]methyl]amino]-5,5,5-trifluoropentanamide, and 80-60 w/v% of HPMC-AS.

14. The composition of claim 13, comprising 25 w/v% of (2R)-2-[[(4-chlorophenyl)sulfonyl][[2-fluoro-4-(1,2,4-oxadiazol-3-yl)phenyl]methyl]amino]-5,5,5-trifluoropentanamide, and 75% of HPMC-AS.

15. The composition of claim 11, wherein said composition is physically and chemically stable for at least 3 months.

16. The composition of claim 15, wherein said composition is physically and chemically stable for at least 6 months.

17. The composition of claim 11, wherein said composition is prepared by spray-drying.

18. The composition of claim 17, wherein said composition is tabletted.

19. The composition of claim 18, wherein said composition is orally bioavailable.

20. The composition of claim 11, wherein said composition is orally bioavailable.

21. A pharmaceutical tablet containing an amorphous solid dispersion composition comprising (2R)-2-[[(4-chlorophenyl)sulfonyl][[[2-fluoro-4-(1,2,4-oxadiazol-3-yl)phenyl]methyl]amino]-5,5,5-trifluoropentanamide and one member selected from the group consisting of PVP-VA and HPMC-AS.

22. A method of preparing an amorphous solid dispersion composition which comprises hot-melt extruding of (2R)-2-[[(4-chlorophenyl)sulfonyl][[2-fluoro-4-(1,2,4-oxadiazol-3-yl)phenyl]methyl]amino]-5,5,5-trifluoropentanamide together with PVP-VA.

23. A method of preparing an amorphous solid dispersion composition which comprises spray drying of (2R)-2-[[(4-chlorophenyl)sulfonyl][[2-fluoro-4-(1,2,4-oxadiazol-3-yl)phenyl]methyl]amino]-5,5,5-trifluoropentanamide together with HPMC-AS.

## Patentansprüche

1. Amorphe feste Dispersionszusammensetzung, umfassend (2R)-2-[[(4-Chlorphenyl)sulfonyl][[2-fluor-4-(1,2,4-oxadiazol-3-yl)phenyl]methyl]amino]-5,5,5-trifluorpentanamid und Polyvinylpyrrolidon-vinylacetat-Copolymer (PVP-VA).

2. Zusammensetzung nach Anspruch 1, umfassend 10-50 Gew.-/Vol.-% (2R)-2-[[(4-Chlorphenyl)sulfonyl][[2-fluor-4-(1,2,4-oxadiazol-3-yl)phenyl]methyl]amino]-5,5,5-trifluorpentanamid und 90-50 Gew.-/Vol.-% PVP-VA.

3. Zusammensetzung nach Anspruch 2, umfassend 20-40 Gew.-/Vol.-% (2R)-2-[[(4-Chlorphenyl)sulfonyl] [[2-fluor-4-(1,2,4-oxadiazol-3-yl)phenyl]methyl]amino]-5,5,5-trifluorpentanamid und 80-60 Gew.-/Vol.-% PVP-VA.

4. Zusammensetzung nach Anspruch 3, umfassend 25 Gew.-/Vol.-% (2R)-2-[[(4-Chlorphenyl)sulfonyl][[2-fluor-4-(1,2,4-oxadiazol-3-yl)phenyl]methyl]amino]-5,5,5-trifluorpentanamid und 75 % PVP-VA.

5. Zusammensetzung nach Anspruch 1, wobei die Zusammensetzung für mindestens 3 Monate physikalisch und chemisch stabil ist.

6. Zusammensetzung nach Anspruch 5, wobei die Zusammensetzung für mindestens 6 Monate physikalisch und chemisch stabil ist.

7. Zusammensetzung nach Anspruch 1, wobei die Zusammensetzung durch Schmelzextrusion oder Sprühtrocknung zubereitet wird.

8. Zusammensetzung nach Anspruch 7, wobei die Zusammensetzung tablettiert wird.

9. Zusammensetzung nach Anspruch 8, wobei die Zusammensetzung oral bioverfügbar ist.

10. Zusammensetzung nach Anspruch 1, wobei die Zusammensetzung oral bioverfügbar ist.

11. Amorphe feste Dispersionszusammensetzung, umfassend (2R)-2-[[(4-Chlorphenyl)sulfonyl][[2-fluor-4-(1,2,4-oxadiazol-3-yl)phenyl]methyl]amino]-5,5,5-trifluorpentanamid und Hydroxypropylmethylcelluloseacetatsuccinat (HPMC-AS).

12. Zusammensetzung nach Anspruch 11, umfassend 10-50 Gew.-/Vol.-% (2R)-2-[[(4-Chlorphenyl)sulfonyl][[2-fluor-4-(1,2,4-oxadiazol-3-yl)phenyl]methyl]amino]-5,5,5-trifluorpentanamid und 90-50 Gew.-/Vol.% HPMC-AS.

13. Zusammensetzung nach Anspruch 12, umfassend 20-40 Gew.-/Vol.-% (2R)-2-[[(4-Chlorphenyl)sulfonyl][[2-fluor-4-(1,2,4-oxadiazol-3-yl)phenyl]methyl]amino]-5,5,5-trifluorpentanamid und 80-60 Gew.-/Vol.-% HPMC-AS.

14. Zusammensetzung nach Anspruch 13, umfassend 25 Gew.-/Vol.-% (2R)-2-[[(4-Chlorphenyl)sulfonyl][[2-fluor-4-(1,2,4-oxadiazol-3-yl)phenyl]methyl]amino]-5,5,5-trifluorpentanamid und 75 % HPMC-AS.

15. Zusammensetzung nach Anspruch 11, wobei die Zusammensetzung für mindestens 3 Monate physikalisch und chemisch stabil ist.

16. Zusammensetzung nach Anspruch 15, wobei die Zusammensetzung für mindestens 6 Monate physikalisch und chemisch stabil ist.

17. Zusammensetzung nach Anspruch 11, wobei die Zusammensetzung durch Sprühtrocknung zubereitet wird.

18. Zusammensetzung nach Anspruch 17, wobei die Zusammensetzung tablettiert wird.

19. Zusammensetzung nach Anspruch 18, wobei die Zusammensetzung oral bioverfügbar ist.

20. Zusammensetzung nach Anspruch 11, wobei die Zusammensetzung oral bioverfügbar ist.

21. Pharmazeutische Tablette, enthaltend eine amorphe feste Dispersionszusammensetzung, umfassend (2R)-2-[[(4-Chlorphenyl)sulfonyl][[2-fluor-4-(1,2,4-oxadiazol-3-yl)phenyl]methyl]amino]-5,5,5-trifluorpentanamid und ein Teil, ausgewählt aus der Gruppe, bestehend aus PVP-VA und HPMC-AS.

22. Verfahren zur Herstellung einer amorphen festen Dispersionszusammensetzung, das Schmelzextrusion von (2R)-2-[[(4-Chlorphenyl)sulfonyl][[2-fluor-4-(1,2,4-oxadiazol-3-yl)phenyl]methyl]amino]-5,5,5-trifluorpentanamid zusammen mit PVP-VA umfasst.

23. Verfahren zur Herstellung einer amorphen festen Dispersionszusammensetzung, das Sprühtrocknung von (2R)-2-[[(4-Chlorphenyl)sulfonyl][[2-fluor-4-(1,2,4-oxadiazol-3-yl)phenyl]methyl]amino]-5,5,5-trifluorpentanamid zusammen mit HPMC-AS umfasst.

## Revendications

1. Composition en dispersion solide amorphe comprenant du (2R)-2-[[(4-chlorophényl)sulfonyl][[2-fluoro-4-(1,2,4-oxadiazol-3-yl)phényl]méthyl]amino]-5,5,5-trifluoropentanamide et un copolymère de polyvinylpyrrolidone-acétate de vinyle (PVP-VA).

2. Composition selon la revendication 1, comprenant 10-50% en poids/volume de (2R)-2-[[(4-chlorophényl)sulfonyl][[2-fluoro-4-(1,2,4-oxadiazol-3-yl)phényl]méthyl]amino]-5,5,5-trifluoropentanamide et 90-50% en poids/volume de PVP-VA.

3. Composition selon la revendication 2, comprenant 20-40% en poids/volume de (2R)-2-[[(4-chlorophényl)sulfonyl][[2-fluoro-4-(1,2,4-oxadiazol-3-yl)phényl]méthyl]amino]-5,5,5-trifluoropentanamide et 80-60% en poids/volume de PVP-VA.

4. Composition selon la revendication 3, comprenant 25% en poids/volume de (2R)-2-[[(4-chlorophényl)sulfonyl][[2-fluoro-4-(1,2,4-oxadiazol-3-yl)phényl]méthyl]amino]-5,5,5-trifluoropentanamide et 75% de PVP-VA.

5. Composition selon la revendication 1, où ladite composition est physiquement et chimiquement stable pendant 3 mois au moins.

6. Composition selon la revendication 5, où ladite composition est physiquement et chimiquement stable pendant 6 mois au moins.

7. Composition selon la revendication 1, où ladite composition est préparée par extrusion par thermofusion ou par séchage par pulvérisation.

8. Composition selon la revendication 7, où ladite composition est mise en comprimés.

9. Composition selon la revendication 8, où ladite composition est biodisponible par voie orale.

10. Composition selon la revendication 1, où ladite composition est biodisponible par voie orale.

11. Composition en dispersion solide amorphe comprenant du (2R)-2-[[(4-chlorophényl)sulfonyl][[2-fluoro-4-(1,2,4-oxadiazol-3-yl)phényl]méthyl]amino]-5,5,5-trifluoropentanamide et d'acétate-succinate d'hydroxypropylméthylcellulose (HPMC-AS).

12. Composition selon la revendication 11, comprenant 10-50% en poids/volume de (2R)-2-[[(4-chlorophényl)sulfonyl][[2-fluoro-4-(1,2,4-oxadiazol-3-yl)phényl]méthyl]amino]-5,5,5-trifluoropentanamide et 90-50% en poids/volume de HPMC-AS.

13. Composition selon la revendication 12, comprenant 20-40% en poids/volume de (2R)-2-[[(4-chlorophényl)sulfonyl][[2-fluoro-4-(1,2,4-oxadiazol-3-yl)phényl]méthyl]amino]-5,5,5-trifluoropentanamide et 80-60% en poids/volume de HPMC-AS.

14. Composition selon la revendication 13, comprenant 25% en poids/volume de (2R)-2-[[(4-chlorophényl)sulfonyl][[2-fluoro-4-(1,2,4-oxadiazol-3-yl)phényl]méthyl]amino]-5,5,5-trifluoropentanamide et 75% de HPMC-AS.

15. Composition selon la revendication 11, où ladite composition est physiquement et chimiquement stable pendant 3 mois au moins.

16. Composition selon la revendication 15, où ladite composition est physiquement et chimiquement stable pendant 6 mois au moins.

17. Composition selon la revendication 11, où ladite composition est préparée par séchage par pulvérisation.

18. Composition selon la revendication 17, où ladite composition est mise en comprimés.

19. Composition selon la revendication 18, où ladite composition est biodisponible par voie orale.

20. Composition selon la revendication 11, où ladite composition est disponible par voie orale.

21. Comprimé pharmaceutique contenant une composition en dispersion solide amorphe comprenant du (2R)-2-[[(4-chlorophényl)sulfonyl][[2-fluoro-4-(1,2,4-oxadiazol-3-yl)phényl]méthyl]amino]-5,5,5-trifluoropentanamide et un membre sélectionné parmi le groupe consistant en PVP-VA et HPMC-AS.

22. Procédé de préparation d'une composition en dispersion solide amorphe comprenant extruder par thermofusion du (2R)-2-[[(4-chlorophényl)sulfonyl][[2-fluoro-4-(1,2,4-oxadiazol-3-yl)phényl]méthyl]amino]-5,5,5-trifluoropentanamide avec du PVP-VA.

23. Procédé de préparation d'une composition en dispersion solide amorphe comprenant sécher par pulvérisation du (2R)-2-[[(4-chlorophényl)sulfonyl][[2-fluoro-4-(1,2,4-oxadiazol-3-yl)phényl]méthyl]amino]-5,5,5-trifluoropentanamide avec du HPMC-AS.
